# EUROPEAN PATENT APPLICATION

(11) **EP 4 806 445 A1**
(43) Date of publication of application: **16.09.2026**
(21) Application number: 24889154.1
(22) Date of filing: 07.11.2024
(51) Int. Cl.: A61K 33/30, A61K 38/05, A61K 45/06, A61P 13/12, A23L 33/16, A23L 33/18

(54) **USE OF COMPOSITION FOR PREVENTING, ALLEVIATING OR TREATING DIABETIC KIDNEY DISEASE COMPRISING ZINC SALT AND CYCLO-HISPRO AS ACTIVE INGREDIENTS**

(30) Priority: 07.11.2023 KR 20230153151
(71) Applicant: Novmetapharma Co., Ltd., Seoul 06050 (KR)
(72) Inventor: JUNG, Hoe Yune, Pohang-si, Gyeongsangbuk-do 37668 (KR); LEE, Do Hyun, Pohang-si, Gyeongsangbuk-do 37667 (KR); JEON, Jongsu, Pohang-si, Gyeongsangbuk-do 37664 (KR); BAEK, Seo Yeong, Pohang-si, Gyeongsangbuk-do 37694 (KR); BAEK, Shin Yu, Pohang-si, Gyeongsangbuk-do 37668 (KR); LEE, Heon Jong, Incheon 21048 (KR)
(74) Representative: Maiwald GmbH
(86) International application number: PCT/KR2024/017546
(87) International publication number: WO 2025/100968

(57) **Abstract**

The present invention relates to a use of a composition for preventing, alleviating, or treating diabetic kidney disease, the composition comprising zinc salt and cyclo-hispro as active ingredients. More specifically, the present invention provides: a composition for preventing, alleviating, or treating diabetic kidney disease, the composition comprising zinc salt and cyclo-hispro as active ingredients, wherein the composition, when administered alone or in combination with an antihypertensive agent and/or antidiabetic agent, exhibits the effect of preventing, alleviating, or treating diabetic kidney disease through the effect of reducing albuminuria, reducing the albuminuria/creatinine ratio, or increasing the glomerular filtration rate; and a method for preventing, alleviating, or treating diabetic kidney disease using same.

## Description

### [TECHNICAL FIELD]

This application claims priority to Korean Patent Application No. 10-2023-0153151, filed on November 7, 2023, the entire disclosure of which is incorporated herein by reference.

The present invention relates to a use of a composition for preventing, alleviating, or treating diabetic kidney disease, the composition comprising zinc salt and cyclo-hispro as active ingredients. More specifically, the present invention provides a composition comprising zinc salt and cyclo-hispro as active ingredients, wherein the composition, when administered alone or in combination with an antihypertensive agent and/or an antidiabetic agent, exhibits the effect of preventing, alleviating, or treating diabetic kidney disease through the effect of reducing albuminuria, reducing the albuminuria/creatinine ratio, or increasing the glomerular filtration rate; and a method for preventing, alleviating, or treating diabetic kidney disease using the same.

### [BACKGROUND ART]

Diabetic kidney disease (DKD) is one of the complications of diabetes, and is known to occur in approximately 40% of diabetic patients (Non-Patent Document 1). Although the pathogenesis of DKD has not yet been clearly elucidated, it is believed to be caused by damage to blood vessels, cells, and tissues in the kidney due to persistent hyperglycemia, hypertension, obesity, high-salt diet, and genetic factors (Non-patent Document 2). Kidney damage caused by DKD causes proteinuria (albuminuria) and reduces the glomerular filtration rate (GFR), which impairs the kidney's important functions of filtration and reabsorption and is a major cause of end-stage renal failure.

Currently, angiotensin receptor blockers (ARBs), such as losartan, which controls blood pressure, and sodium glucose cotransporter 2 (SGLT2) inhibitors, such as dapagliflozin, which controls blood sugar, are used for the prevention and treatment of DKD. However, it is reported that the losartan, which is classified as an angiotensin II receptor blocker, may cause common side effects such as indigestion, diarrhea, and abdominal pain, and the dapagliflozin, which has the most clinical results among the SGLT2 inhibitors, commonly causes side effects such as headache, diarrhea, back pain, bronchitis, laryngopharyngitis, and upper respiratory tract infections. Moreover, there is a problem that routine treatments of blood pressure and blood sugar control alone are unlikely to be effective enough to slow or halt the progression of DKD. Therefore, there is a need for the strategic development of drug that enhances the level of therapeutic efficacy through new drugs or synergistic effects due to combinations of existing drugs.

Meanwhile, Patent Document 1 discloses a composition including zinc ions and cyclo-hispro (CHP) as a composition useful for alleviating diabetic symptoms in mammals, but fails to disclose examples of combining the composition with an antihypertensive agent and/or an antidiabetic agent for the purpose of preventing, alleviating, or treating the DKD.

Against this background, the present inventors have confirmed that the combination of zinc and cyclo-hispro not only exhibits the effect of preventing, alleviating, or treating diabetic kidney disease through the effects of reducing albuminuria, reducing the albuminuria/creatinine ratio, or increasing the glomerular filtration rate, but also significantly enhances the DKD treatment effect of existing antihypertensive and/or antidiabetic agents prescribed to DKD patients when administered in combination with such agents, thereby completing the present invention.

### [Prior Art Literature]

### [Patent Documents]

[Patent Document 1] Korean Patent Laid-Open Publication No. 10-2001-0022786.

### [Non-patent Documents]

[Non-patent Document 1] Am Soc Nephrol : CJASN., 12 (2017), pp. 2032-204

[Non-patent Document 2] Mol Sci. 2020 Mar 23;21(6):2218

### [DISCLOSURE]

### [TECHNICAL PROBLEM]

Accordingly, the present invention aims to provide a composition for preventing, alleviating, or treating diabetic kidney disease, comprising a combination of zinc and cyclo-hispro.

Another object of the present invention is to provide a composition for enhancing the efficacy of an antihypertensive agent and/or an antidiabetic agent in the treatment of diabetic kidney disease, comprising a combination of zinc and cyclo-hispro.

In addition, the present invention aims to provide a method for preventing, alleviating, or treating diabetic kidney disease using a combination of zinc and cyclo-hispro.

Furthermore, the present invention aims to provide a method for enhancing the efficacy of an antihypertensive agent and/or an antidiabetic agent in preventing, alleviating, or treating diabetic kidney disease by using a combination of zinc and cyclo-hispro.

Additionally, the present invention aims to provide a use of a composition comprising a combination of zinc and cyclo-hispro for the manufacture of a medicament for preventing, alleviating, or treating diabetic kidney disease.

In addition, the present invention aims to provide a use of a composition comprising a combination of zinc and cyclo-hispro for the manufacture of a medicament for enhancing the efficacy of an antihypertensive agent and/or an antidiabetic agent in preventing, alleviating, or treating diabetic kidney disease.

### [TECHNICAL SOLUTION]

In order to achieve the above-described objects, the present invention provides a pharmaceutical composition for preventing or treating diabetic kidney disease, the composition comprising, as active ingredients, a zinc salt; and cyclo-hispro or a pharmaceutically acceptable salt thereof.

Further, the present invention provides a pharmaceutical composition for enhancing the efficacy of an antihypertensive agent and/or an antidiabetic agent in the treatment of diabetic kidney disease, the composition comprising, as active ingredients, a zinc salt; and cyclo-hispro or a pharmaceutically acceptable salt thereof.

In addition, the present invention provides a health functional food composition for preventing or alleviating diabetic kidney disease, the composition comprising, as active ingredients, a zinc salt; and cyclo-hispro or a food-acceptable salt thereof.

In the present invention, the pharmaceutical composition may further comprise, as an additional active ingredient, an antihypertensive agent selected from the group consisting of angiotensin II receptor blockers (ARBs) and angiotensin-converting-enzyme inhibitors (ACE inhibitors).

In the present invention, the pharmaceutical composition may comprise, as an additional active ingredient, an antidiabetic agent selected from the group consisting of sodium glucose cotransporter 2 inhibitors (SGLT2 inhibitors), dipeptidyl peptidase-4 inhibitors (DPP-4 inhibitors), and biguanide drugs.

In the present invention, the pharmaceutical composition may comprise, as additional active ingredients, an antihypertensive agent selected from the group consisting of ARB and ACE inhibitors, and an antidiabetic agent selected from the group consisting of SGLT2 inhibitors, DPP-4 inhibitors, and biguanide drugs.

In the present invention, the ARB may be any one selected from the group consisting of losartan, valsartan, candesartan, olmesartan, telmisartan, fimasartan, irbesartan, eprosartan, and azilsartan, and the ACE inhibitor may be any one selected from the group consisting of captopril, zofenopril, enalapril, ramipril, quinapril, perindopril, lisinopril, benazepril, imidapril, trandolapril, cilazapril, and fosinopril.

In the present invention, the SGLT2 inhibitor may be any one selected from the group consisting of dapagliflozin, canagliflozin, empagliflozin, ipragliflozin, tofogliflozin, luseogliflozin, remogliflozin, remogliflozin etabonate, and ertugliflozin; the DPP-4 inhibitor may be any one selected from the group consisting of sitagliptin, linagliptin, vildagliptin, gemigliptin, saxagliptin, alogliptin, teneligliptin, anagliptin, and evogliptin; and the biguanide drug may be any one selected from the group consisting of metformin, buformin, and phenformin.

In the present invention, the composition may exhibit at least one effect selected from the group consisting of i) reducing albuminuria; ii) reducing albuminuria/creatinine ratio; and iii) increasing glomerular filtration rate.

In the present invention, the composition may be administered simultaneously, separately, or sequentially with the antihypertensive agent and/or the antidiabetic agent.

In the present invention, the health functional food composition may be for enhancing the effect of alleviating diabetic kidney disease in a DKD patient receiving the antihypertensive agent and/or the antidiabetic agent.

Additionally, the present invention provides a method for preventing, alleviating, or treating diabetic kidney disease, the method comprising administering to a subject in need thereof an effective amount of a composition comprising a zinc salt and cyclo-hispro or a pharmaceutically acceptable salt thereof.

In addition, the present invention provides a method for enhancing the efficacy of an antihypertensive agent and/or an antidiabetic agent in the treatment of diabetic kidney disease, the method comprising administering to a subject in need thereof an effective amount of a composition comprising a zinc salt and cyclo-hispro or a pharmaceutically acceptable salt thereof.

Furthermore, the present invention provides a use of a composition comprising a zinc salt; and cyclo-hispro or a pharmaceutically acceptable salt thereof for the manufacture of a medicament for preventing, alleviating, or treating diabetic kidney disease.

In addition, the present invention provides a use of a composition comprising a zinc salt; and cyclo-hispro or a pharmaceutically acceptable salt thereof for the manufacture of a medicament for enhancing the efficacy of an antihypertensive agent and/or an antidiabetic agent in preventing, alleviating, or treating diabetic kidney disease.

In the present invention, the composition may further comprise, as an additional active ingredient, an antihypertensive agent selected from the group consisting of angiotensin II receptor blockers (ARBs) and angiotensin-converting-enzyme inhibitors (ACE inhibitors), or may be administered in combination with the antihypertensive agent.

In the present invention, the composition may comprise, as an additional active ingredient, an antidiabetic agent selected from the group consisting of sodium glucose cotransporter 2 inhibitors (SGLT2 inhibitors), dipeptidyl peptidase-4 inhibitors (DPP-4 inhibitors), and biguanide drugs, or may be administered in combination with the antidiabetic agent.

In the present invention, the composition may comprise, as additional active ingredients, an antihypertensive agent selected from the group consisting of ARB and ACE inhibitors, and an antidiabetic agent selected from the group consisting of SGLT2 inhibitors, DPP-4 inhibitors, and biguanide drugs, or may be administered in combination with the antihypertensive agent and the antidiabetic agent.

In the present invention, the ARB may be any one selected from the group consisting of losartan, valsartan, candesartan, olmesartan, telmisartan, fimasartan, irbesartan, eprosartan, and azilsartan, and the ACE inhibitor may be any one selected from the group consisting of captopril, zofenopril, enalapril, ramipril, quinapril, perindopril, lisinopril, benazepril, imidapril, trandolapril, cilazapril, and fosinopril.

In the present invention, the SGLT2 inhibitor may be any one selected from the group consisting of dapagliflozin, canagliflozin, empagliflozin, ipragliflozin, tofogliflozin, luseogliflozin, remogliflozin, remogliflozin etabonate, and ertugliflozin; the DPP-4 inhibitor may be any one selected from the group consisting of sitagliptin, linagliptin, vildagliptin, gemigliptin, saxagliptin, alogliptin, teneligliptin, anagliptin, and evogliptin; and the biguanide drug may be any one selected from the group consisting of metformin, buformin, and phenformin.

In the present invention, the composition may exhibit at least one effect selected from the group consisting of i) reducing albuminuria; ii) reducing albuminuria/creatinine ratio; and iii) increasing glomerular filtration rate.

In the present invention, the composition may be administered simultaneously, separately, or sequentially with the antihypertensive agent and/or the antidiabetic agent.

### [ADVANTAGEOUS EFFECTS]

The composition comprising zinc salt and cyclo-hispro according to the present invention exhibits an effect of preventing, alleviating, or treating diabetic kidney disease through the effect of reducing albuminuria, reducing the albuminuria/creatinine ratio or increasing the glomerular filtration rate when the combination is administered alone, and thus can be utilized as a therapeutic agent and a health functional food for preventing, alleviating, or treating diabetic kidney disease. Furthermore, when administered in combination with antihypertensive and/or antidiabetic agents that are conventionally prescribed to patients with diabetic kidney disease, the composition significantly enhances the efficacy of these agents in the treatment of diabetic kidney disease, and therefore, is useful as an adjuvant for patients receiving these drugs, and may also contribute to the reduction of side effects caused by the chronic administration of these drugs.

### [DESCRIPTION OF DRAWINGS]

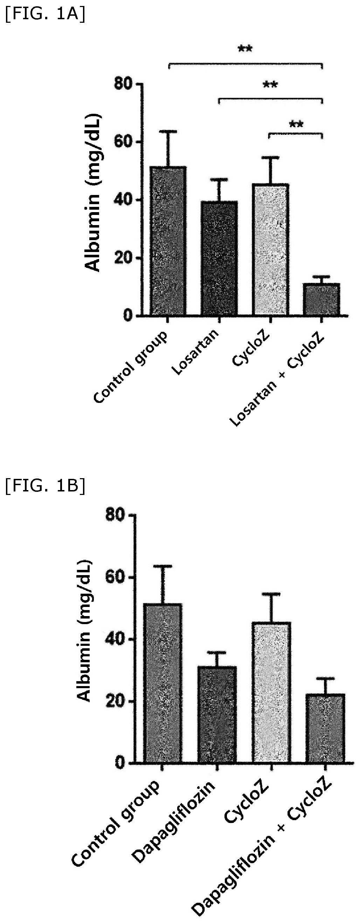

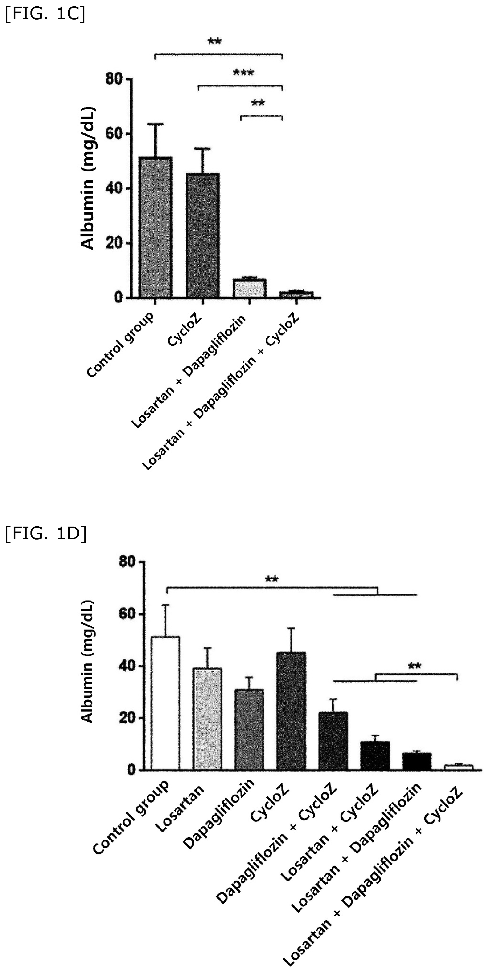

FIGS. 1A to 1D show the results of confirming the effect of reducing albuminuria according to the administration of CycloZ or various drug combinations in KKAy mice, a diabetic kidney disease model, wherein FIG. 1A shows the albuminuria reduction effect by administering CycloZ alone or a combination of losartan + CycloZ; FIG. 1B shows the albuminuria reduction effect by administering CycloZ alone or a combination of dapagliflozin and CycloZ; FIG. 1C shows the albuminuria reduction effect by administering CycloZ alone or a combination of losartan, dapagliflozin, and CycloZ; and FIG. 1D shows one graph comparing the results of FIGS. 1A to 1C.

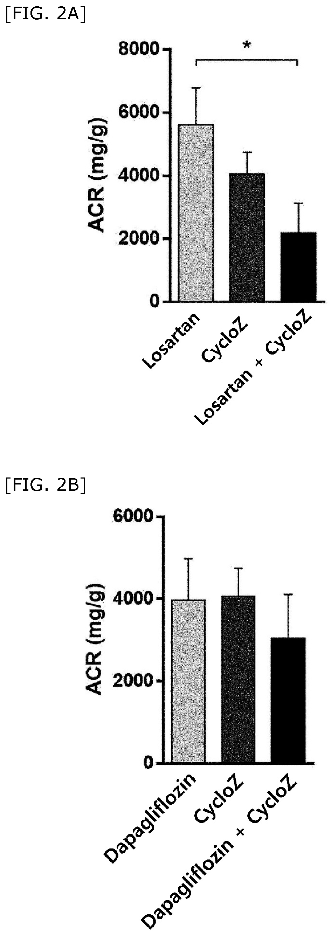

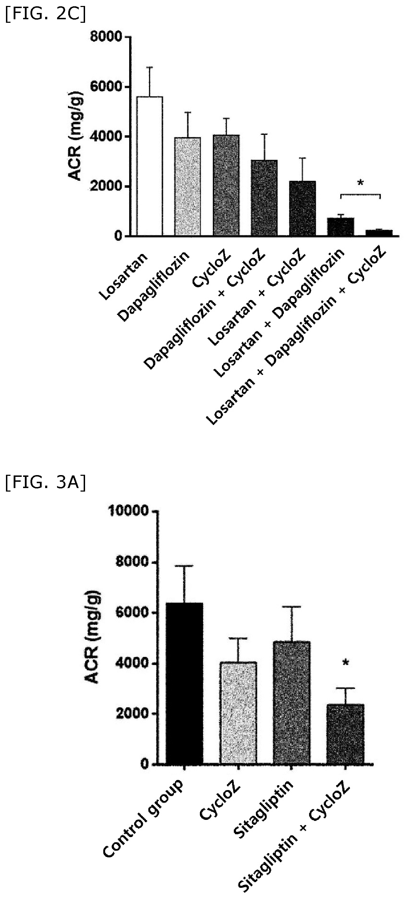

FIGS. 2A to 2C show the results of confirming the effect of reducing the albuminuria/creatinine ratio (Urine Albumin to Creatinine Ratio, ACR) according to the administration of CycloZ or various drug combinations in KKAy mice, a diabetic kidney disease model, wherein FIG. 2A shows the ACR reduction effect by administering CycloZ alone or a combination of losartan and CycloZ; FIG. 2B shows the ACR reduction effect by administering CycloZ alone or a combination of dapagliflozin and CycloZ; and FIG. 2C shows the ACR reduction effect by administering CycloZ alone or a combination of losartan, dapagliflozin, and CycloZ.
FIG. 3A shows the ACR reduction effect by administering CycloZ alone, sitagliptin alone, or a combination of sitagliptin and CycloZ starting from the mild stage in KKAy mice, a diabetic kidney disease model.
FIG. 3B shows the ACR changes by administering CycloZ alone, sitagliptin alone, or a combination of sitagliptin and CycloZ starting from the severe stage in KKAy mice, a diabetic kidney disease model.
FIG. 4 shows the ACR changes by administering CycloZ alone, losartan alone, a combination of losartan and CycloZ, or a combination of losartan and sitagliptin starting from the severe stage in KKAy mice, a diabetic kidney disease model.
FIG. 5 shows the ACR changes by administering CycloZ alone, metformin alone, or a combination of metformin and CycloZ starting from the severe stage in KKAy mice, a diabetic kidney disease model.
FIG. 6 shows the ACR reduction effect by CHP alone and a combination of zinc salt and CHP in KKAy mice, a diabetic kidney disease model.

### [BEST MODES OF THE INVENTION]

Hereinafter, the present invention will be described in more detail.

All technical terms used in the present invention, unless otherwise defined, are used in the same sense as commonly understood by those skilled in the art to which the invention pertains. In addition, although preferred methods or samples are described herein, similar or equivalent methods and samples are also included within the scope of the present invention.

As described above, it is difficult to expect effective treatment, such as slowing or halting the progression of diabetic kidney disease, with existing therapeutic agents for diabetic kidney disease. Accordingly, the present inventors have sought a solution to the above-mentioned problem by experimentally verifying that the combination of zinc and cyclo-hispro not only exhibits the effect of preventing, alleviating, or treating diabetic kidney disease through the effects of reducing albuminuria, reducing the albuminuria/creatinine ratio, or increasing the glomerular filtration rate, but also significantly enhances the DKD treatment effect of existing antihypertensive and/or antidiabetic agents prescribed to DKD patients when administered in combination with such agents.

Accordingly, in a first aspect, the present invention relates to a pharmaceutical composition for preventing or treating diabetic kidney disease, the composition comprising, as active ingredients, a zinc salt; and cyclo-hispro or a pharmaceutically acceptable salt thereof.

In relation to the first aspect, the present invention provides a method for preventing, alleviating, or treating diabetic kidney disease, the method comprising administering to a subject in need thereof an effective amount of a composition comprising a zinc salt and cyclo-hispro or a pharmaceutically acceptable salt thereof.

In connection with the first aspect, the present invention further provides a use of a composition comprising a zinc salt; and cyclo-hispro or a pharmaceutically acceptable salt thereof for preventing, alleviating, or treating diabetic kidney disease.

In connection with the first aspect, the present invention also provides a use of a composition comprising a zinc salt; and cyclo-hispro or a pharmaceutically acceptable salt thereof for the manufacture of a medicament for preventing, alleviating, or treating diabetic kidney disease.

In the present invention, the zinc salt may be a zinc salt selected from the group consisting of zinc ion, zinc metal, zinc chloride, zinc acetate, zinc gluconate, zinc stearate, zinc sulfate, zinc oxide, zinc picolinate, zinc orotate, and zinc citrate, but is not limited thereto.

In the present invention, the "cyclo-hispro (CHP)" refers to a naturally occurring cyclic dipeptide composed of histidine-proline, which is a metabolite of thyrotropin-releasing hormone (TRH), or a physiologically active dipeptide that is synthesized de novo in the body through the TRH metabolic process, and is widely distributed throughout the brain, spinal cord, and gastrointestinal tract.

In the present invention, the cyclo-hispro may be either synthesized or commercially available. Furthermore, it may be purified from materials containing cyclo-hispro, such as prostate extracts and soybean hydrolysates.

By the use of the term "purified", it is intended to mean that the cyclo-hispro is in a concentrated form compared to a form obtainable from natural sources such as prostate extract. The purified ingredient may be obtained either by concentrating from a natural source or through a chemical synthesis method.

In the present invention, the zinc salt and cyclo-hispro are also referred to as "cyclo-Z." The zinc salt and cyclo-hispro may be included in the composition of the present invention either in the form of a composite or as separate ingredients. In this case, the weight ratio of zinc salt to cyclo-His-Pro may be 1-10 : 1-5, and preferably 1-5 : 1-2, but is not limited thereto. In the present invention, the weight ratio for zinc salt represents the weight ratio for the amount of zinc cations or the zinc component in the zinc salt.

Therefore, the zinc salt and cyclo-hispro may be administered in the form of a single composite, or administered as individual ingredients simultaneously, separately or sequentially.

In the present invention, the pharmaceutical composition may further include an antihypertensive agent as another effective ingredient.

Additionally, in the present invention, the composition administered to the subject may be administered in combination with the antihypertensive agent.

In the present invention, the antihypertensive agent may be an ARB or an ACE inhibitor. The ARB and ACE are antihypertensive agents that inhibit the action of the renin-angiotensin-aldosterone system (RAAS), wherein the ARB blocks angiotensin II from acting on angiotensin II receptor type 1 (AT1), and ACE inhibitors inhibit ACE to block the conversion of angiotensin I to angiotensin II.

In the present invention, the ARB may be any one selected from the group consisting of losartan, valsartan, candesartan, olmesartan, telmisartan, fimasartan, irbesartan, eprosartan, and azilsartan, but is not limited thereto.

In the present invention, the ACE inhibitor may be any one selected from the group consisting of captopril, zofenopril, enalapril, ramipril, quinapril, perindopril, lisinopril, benazepril, imidapril, trandolapril, cilazapril, and fosinopril, but is not limited thereto.

In the present invention, the weight ratio of the zinc salt and cyclo-hispro (Cyclo-Z) to the antihypertensive agent may be 1 to 2 : 1 to 4, wherein the antihypertensive agent may be at a concentration equal to or higher than that of the zinc salt and cyclo-hispro (Cyclo-Z), but is not limited thereto.

The angiotensin II is a major end product of the renin-angiotensin system (RAS), and is known to be a mediator that plays a central role in the development or progression of cardiovascular disease at various points in the cardiovascular disease continuum by binding to AT1 receptors to cause effects such as vasoconstriction, increased sodium absorption in the renal tubules, water retention, decreased endothelial function, increased oxidative stress, and increased tissue fibrosis and collagen deposition. The angiotensin II receptor blockers are currently the most widely used drugs for the treatment of hypertension due to their excellent hypotensive effect and few side effects through physiological blockade of the AT1 receptor of the RAS. However, despite these various benefits, concerns are raised about the safety of ARBs, such as the so-called ARB-myocardial infarction paradox, which suggests that the use of ARBs may rather increase myocardial infarction, and the controversy over increased incidence of cancer.

In the present invention, the pharmaceutical composition may further include an antidiabetic agent as still another effective ingredient.

Additionally, in the present invention, the composition administered to the subject may be administered in combination with the antidiabetic agent.

In the present invention, the antidiabetic agent may be any one selected from the group consisting of an SGLT2 inhibitor, a DPP-4 inhibitor, and a biguanide drug.

In the present invention, the SGLT2 inhibitor may be any one selected from the group consisting of dapagliflozin, canagliflozin, empagliflozin, ipragliflozin, tofogliflozin, luseogliflozin, remogliflozin, remogliflozin etabonate, and ertugliflozin, but is not limited thereto.

In the present invention, the DPP-4 inhibitor may be any one selected from the group consisting of sitagliptin, linagliptin, vildagliptin, gemigliptin, saxagliptin, alogliptin, teneligliptin, anagliptin, and evogliptin, but is not limited thereto.

The biguanide drug may be any one selected from the group consisting of metformin, buformin, and phenformin.

In the present invention, the weight ratio of the zinc salt and cyclo-hispro (Cyclo-Z) to the SGLT2 inhibitor may be 5 to 10 : 1 to 1.5, but is not limited thereto.

In the present invention, the weight ratio of the zinc salt and cyclo-hispro (Cyclo-Z) to the DPP-4 inhibitor may be 1 to 3.5 : 1 to 2, wherein the DPP-4 inhibitor may be at a concentration equal to or lower than that of the zinc salt and cyclo-hispro (Cyclo-Z), but is not limited thereto.

In the present invention, the weight ratio of the zinc salt and cyclo-hispro (Cyclo-Z) to the biguanide drug may be 1 to 2 : 10 to 16, but is not limited thereto.

In the present invention, the pharmaceutical composition may further include both the antihypertensive agent and the antidiabetic agent as other effective ingredients, wherein the description of the antihypertensive agent and the antidiabetic agent is the same as described above, and thus is omitted.

In addition, in the present invention, the composition administered to the subject may be administered in combination with the antihypertensive agent and the antidiabetic agent, wherein the description of the antihypertensive agent and the antidiabetic agent is the same as described above, and thus is omitted.

In the present invention, when the pharmaceutical composition comprises zinc salt and cyclo-hispro (Cyclo-Z), an antihypertensive agent, and an SGLT2 inhibitor, the weight ratio of the zinc salt and cyclo-hispro (Cyclo-Z) : antihypertensive agent : SGLT2 inhibitor may be 5 to 10 : 5 to 10 : 0.5 to 1, wherein the antihypertensive agent may be at a concentration equal to or higher than that of the zinc salt and cyclo-hispro (Cyclo-Z), but is not limited thereto.

In the present invention, when the pharmaceutical composition comprises zinc salt and cyclo-hispro (Cyclo-Z), an antihypertensive agent, and a DPP-4 inhibitor, the weight ratio of the zinc salt and cyclo-hispro (Cyclo-Z) : antihypertensive agent : DPP-4 inhibitor may be 5 to 10 : 5 to 10 : 2 to 5, wherein the antihypertensive agent may be at a concentration equal to or higher than that of the zinc salt and cyclo-hispro (Cyclo-Z), and the DPP-4 inhibitor may be at a concentration lower than that of the zinc salt and cyclo-hispro (Cyclo-Z) and the antihypertensive agent, but is not limited thereto.

In the present invention, the zinc salt and cyclo-hispro (Cyclo-Z) may be included in a single composite form at a dose of 15 to 100 mg/kg when applied clinically, for example, at a dose of 38 mg/kg, 76 mg/kg, or 91 mg/kg per dosage unit, but not limited to. When the zinc salt and cyclo-hispro are included as separate ingredients, they may be included in a dose calculated according to the weight ratio of zinc salt: cyclo-hispro defined in the present invention within the above dose range.

In the present invention, the SGLT2 inhibitor may be included at a dose of 2 to 15 mg/kg when applied clinically, for example, at a dose of 5 mg/kg or 10 mg/kg per dosage unit, but not limited to.

In the present invention, the DPP-4 inhibitor may be included at a dose of 10 to 120 mg/kg when applied clinically. For example, it may be included at a dose of 25 mg/kg, 50 mg/kg, or 100 mg/kg per dosage unit, but not limited to.

In the present invention, the biguanide drug may be included at a dose of 200 to 1500 mg/kg when applied clinically. For example, it may be included at a dose of 500 mg/kg or 1000 mg/kg per dosage unit, but not limited to.

As side effects of existing antihypertensive and antidiabetic agents have been reported, the present inventors have sought to find a substance that could prevent or reduce side effects resulting from overdose or long-term administration of antihypertensive and/or antidiabetic agents by replacing the combined administration of these agents or by significantly increasing the therapeutic effect of these agents on diabetic kidney disease. As a result, as confirmed in FIGS. 1A to 1D, it was confirmed that the combination of zinc salt and cyclo-hispro not only showed an albuminuria reduction effect in the KKay mouse model of diabetic kidney disease, but also significantly increased the albuminuria reduction effect of losartan, a representative ARB drug, and dapagliflozin, a representative SGLT2 drug when administered in combination with the losartan and/or the dapagliflozin.

In addition, as confirmed in FIGS. 2A to 2C, it was confirmed that the combination of zinc salt and cyclo-hispro (CycloZ) not only showed an ACR reduction effect in the KKay mouse model of diabetic kidney disease, but also significantly increased the ACR reduction effect of losartan and dapagliflozin when administered in combination with the losartan and/or the dapagliflozin.

Additionally, as confirmed in Table 2, it was confirmed that in the KKay mouse model of diabetic kidney disease, the combination of zinc salt and cyclo-hispro was administered in combination with losartan and/or dapagliflozin, thereby further increasing the glomerular filtration rate-increasing effect of these drugs.

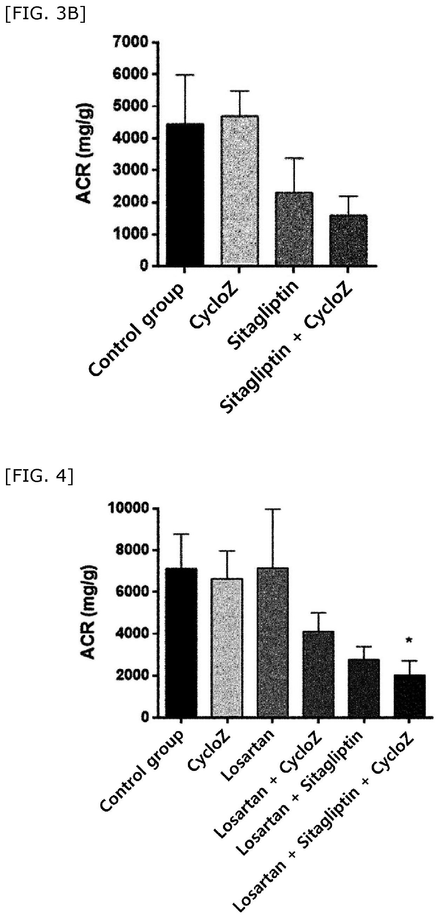

In addition, as confirmed in FIGS. 3A and 3B, it was confirmed that in the KKay mouse model of diabetic kidney disease, when administered in combination with sitagliptin, a representative drug of DPP-4 inhibitors, the combination of zinc salt and cyclo-hispro significantly increased the ACR reduction effect compared to the administration of sitagliptin alone, regardless of the severity of diabetic kidney disease.

Furthermore, as confirmed in FIG. 4, it was confirmed that in the KKay mouse model of diabetic kidney disease, when administered in combination with losartan, a representative ARB drug, and sitagliptin, a representative DPP-4 inhibitor drug, the combination of cyclo-hispro significantly increased the ACR reduction effect compared to the co-administration of the losartan and sitagliptin.

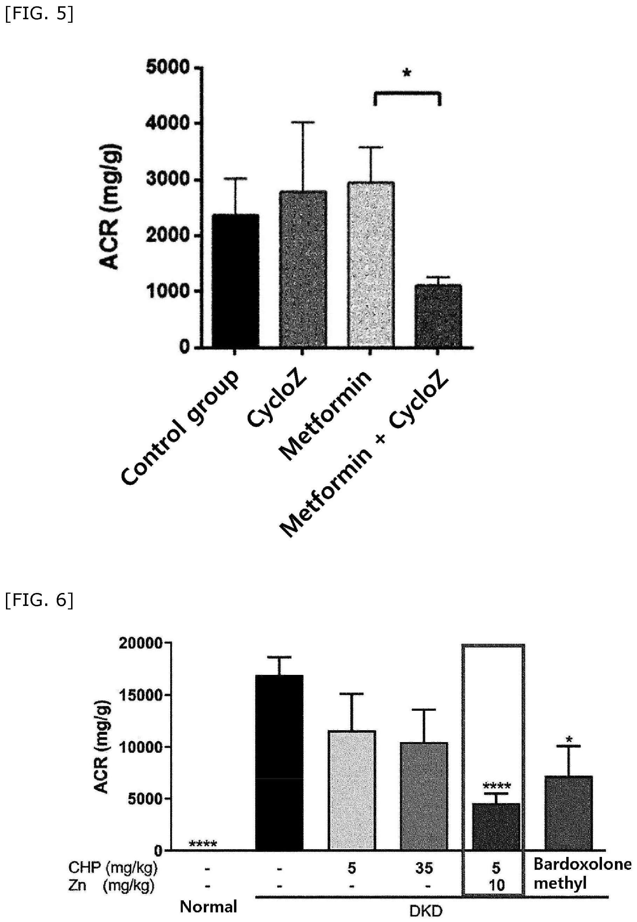

In addition, as confirmed in FIG. 5, it was confirmed that in the KKay mouse model of diabetic kidney disease, when administered in combination with metformin, a representative drug of biguanides, the combination of cyclo-hispro significantly increased the ACR reduction effect compared to the administration of the cyclo-hispro alone and the metformin alone.

Additionally, as confirmed in FIG. 6, it was confirmed that in the KKay mouse model of diabetic kidney disease, the combination of zinc salt and cyclo-hispro (CycloZ) significantly reduced the ACR level compared to the cyclo-hispro alone, exhibiting a synergistic effect of co-administration of the zinc salt and cyclo-hispro.

Accordingly, the pharmaceutical composition of the present invention can exhibit at least one effect selected from the group consisting of i) reducing albuminuria; ii) reducing albuminuria/creatinine ratio; and iii) increasing glomerular filtration rate.

In the present invention, the diabetic kidney disease includes both type 1 diabetic kidney disease and type 2 diabetic kidney disease, and the effects of i) to iii) above can be equally exhibited regardless of the type of diabetes.

The combination of zinc salt and cyclo-hispro is known to exert a therapeutic effect by reducing blood sugar in type 2 diabetes, which is a non-insulin dependent diabetes, but it does not show a clear therapeutic effect, such as reducing blood sugar, in type 1 diabetes, which is an insulin dependent diabetes. However, in the case of diabetic kidney disease, the combination exhibits the effects of reducing albuminuria, reducing the albuminuria/creatinine ratio, and increasing the glomerular filtration rate (GFR) regardless of the type of diabetes (i.e., in both type 1 and type 2 diabetes), and thus exhibits the effects of preventing, alleviating, or treating diabetic kidney disease through an independent activity separate from the effects of treating type 2 diabetes.

In the present invention, the pharmaceutical composition may be administered simultaneously, separately, or sequentially with the antihypertensive agent and/or the antidiabetic agent.

In the present invention, the term "synergistic effect" refers to an effect that occurs when individual ingredients are co-administered (administered in combination), which is greater than the sum of the effects that occur when each ingredient is administered alone. The composition comprising a zinc salt and cyclo-hispro or a pharmaceutically acceptable salt thereof according to the present invention increases the effect of preventing, alleviating, or treating diabetic kidney disease by being administered in combination with an antihypertensive agent and/or an antidiabetic agent.

The term "administered in combination" or "co-administered" means that compounds or ingredients are administered together to a patient. The fact that each compound or ingredient is administered together means that, in order to achieve the desired therapeutic effect, each ingredient may be administered at the same time, in any order, or sequentially at different times.

The term "prevention" as used herein refers to any act of inhibiting or delaying the onset of a disease or pathological condition. In the present invention, it means delaying the onset of diabetic kidney disease or suppressing its onset.

The term "alleviation" as used herein refers to any act of ameliorating or beneficially changing the state of a disease or pathological condition, and in the present invention means ameliorating the symptoms of diabetic kidney disease.

The term "treatment" as used herein refers to any act of delaying, stopping, or reversing the progression of a disease or pathological condition, and in the present invention means alleviating, mitigating, eliminating, or reversing the symptoms of diabetic kidney disease.

The term "pharmaceutically acceptable salt" as used herein refers to any organic or inorganic addition salt of cyclo-hispro that is relatively non-toxic to patients, has a harmless effective action, and does not diminish the beneficial effects of cyclo-hispro due to side effects caused by the salt. For these salts, inorganic and organic acids may be used as free acids, wherein the inorganic acids may include hydrochloric acid, hydrobromic acid, nitric acid, sulfuric acid, perchloric acid, phosphoric acid, etc., and the organic acids may include citric acid, acetic acid, lactic acid, maleic acid, fumaric acid, gluconic acid, methanesulfonic acid, glycolic acid, succinic acid, tartaric acid, galacturonic acid, embonic acid, glutamic acid, aspartic acid, oxalic acid, (D) or (L) malic acid, maleic acid, methanesulfonic acid, ethanesulfonic acid, 4-toluenesulfonic acid, salicylic acid, citric acid, benzoic acid, malonic acid, etc. Additionally, these salts include alkali metal salts (sodium salts, potassium salts, etc.) and alkaline earth metal salts (calcium salts, magnesium salts, etc.). For example, acid addition salts may include acetate, aspartate, benzate, besylate, bicarbonate/carbonate, bisulfate/sulfate, borate, camsylate, citrate, edisylate, esylate, formate, fumarate, gluceptate, gluconate, glucuronate, hexafluorophosphate, hybenzate, hydrochloride/chloride, hydrobromide/bromide, hydroiodide/iodide, isethionate, lactate, malate, maleate, malonate, mesylate, methyl sulfate, naphthylate, 2-naphthylate, nicotinate, nitrate, orotate, oxalate, palmitate, pamoate, phosphate/hydrogen phosphate/dihydrogen phosphate, saccharate, stearate, succinate, tartrate, tosylate, trifluoroacetate, aluminum, arginine, benzathine, calcium, choline, diethylamine, diolamine, glycine, lysine, magnesium, meglumine, olamine, potassium, sodium, tromethamine, zinc salts, etc..

The pharmaceutical composition of the present invention may additionally comprise a pharmaceutically acceptable carrier. The pharmaceutical composition comprising a pharmaceutically acceptable carrier may be in various oral or parenteral dosage forms. When formulated, it is prepared using commonly used diluents or excipients such as fillers, extenders, binders, wetting agents, disintegrants, and surfactants. Solid formulations for oral administration may include tablets, pills, powders, granules, capsules, troches, etc., wherein these solid formulations may be prepared by mixing one or more compounds of the present invention with at least one excipient, such as starch, calcium carbonate, sucrose or lactose, or gelatin. Additionally, in addition to simple excipients, lubricants such as magnesium stearate and talc may also be used. Liquid formulations for oral administration may include suspensions, internal solutions, emulsions, syrups, etc., which may contain various excipients such as wetting agents, sweeteners, fragrances, and preservatives in addition to commonly used simple diluents such as water and liquid paraffin.

Formulations for parenteral administration may include sterile aqueous solutions, non-aqueous solutions, suspensions, emulsions, lyophilized preparations, suppositories, etc. As non-aqueous solvents and suspending agents, propylene glycol, polyethylene glycol, vegetable oils such as olive oil, and injectable esters such as ethyl oleate may be used. As the base of the suppository, witepsol, macrogol, Tween 61, cacao butter, laurin butter, glycerol, and gelatin, etc. may be used.

The pharmaceutical composition of the present invention can be administered via any conventional route capable of delivering the composition to the target tissue or cells within a subject or sample. The administration may include systemic or local administration, and may include intraperitoneal administration, intravenous administration, intramuscular administration, subcutaneous administration, intradermal administration, oral administration, intranasal administration, intrapulmonary administration, and rectal administration, but is not limited thereto. The dosage of the pharmaceutical composition may vary depending on the patient's age, weight, sex, dosage form, health condition, and disease severity.

In the method of the present invention, the term "subject" includes any animal (e.g., human, horse, pig, rabbit, dog, sheep, goat, non-human primate, cow, cat, guinea pig, or rodent), but is not limited thereto. These terms do not indicate a specific age or sex. Thus, it is intended to include adult/mature and neonatal subjects, as well as fetuses, regardless of whether they are female or male. A patient refers to a subject suffering from a disease or disorder. The term "patient" includes human and veterinary subjects.

In the method of the present invention, the description of the constitution including the effects, administration route, number of administrations, dosage, etc. of the zinc salt and cyclo-hispro or a pharmaceutically acceptable salt thereof administered to the subject is the same as described above, and therefore, is omitted.

In the method of the present invention, the zinc salt and cyclo-hispro can provide a desirable effect of preventing, alleviating, or treating diabetic kidney disease when administered in an effective amount. For a desirable effect, the combination of the zinc salt and cyclo-hispro or a pharmaceutically acceptable salt thereof according to the present invention may be administered once or repeatedly several times at regular time intervals. In this case, the zinc salt and the cyclo-hispro may be administered simultaneously, separately, or sequentially. For example, the zinc salt and cyclo-hispro or a pharmaceutically acceptable salt thereof may be co-formulated and administered simultaneously as a combined unit dosage form, or may be administered simultaneously or sequentially as separate formulations.

Alternatively, a composition comprising the zinc salt and cyclo-hispro can provide a desirable effect of preventing, alleviating, or treating diabetic kidney disease when administered in an effective amount in combination with an antihypertensive agent and/or an antidiabetic agent. For a desirable effect, the composition comprising the zinc salt and cyclo-hispro or a pharmaceutically acceptable salt thereof of the present invention in combination with the antihypertensive and/or antidiabetic agents may be administered once or repeatedly several times at regular time intervals. In this case, the composition comprising the zinc salt and cyclo-hispro or a pharmaceutically acceptable salt thereof of the present invention in combination with the antihypertensive and/or antidiabetic agents may be administered simultaneously, separately, or sequentially. For example, the composition comprising the zinc salt and cyclo-hispro or a pharmaceutically acceptable salt thereof in combination with the antihypertensive and/or antidiabetic agents may be co-formulated and administered simultaneously as a combined unit dosage form, or may be administered simultaneously or sequentially as separate formulations.

When administered sequentially, each active ingredient may be administered as an individual formulation at time intervals, and the order of administration may be determined by a physician or a person having ordinary knowledge in the field.

Additionally, it may be used in combination with other methods to prevent, alleviate, or treat diabetic kidney disease.

In the use of the present invention, the description of the constitution including the effects, administration route, number of administrations, dosage, etc. of the composition including the zinc salt and cyclo-hispro or a pharmaceutically acceptable salt thereof is the same as described above, and therefore, is omitted.

The composition comprising a zinc salt and cyclo-hispro or a pharmaceutically acceptable salt thereof as active ingredients according to the present invention significantly increases the effect of treating diabetic kidney disease when administered in combination with an ARB and/or an SGLT2 inhibitor, and thus can also be utilized as an adjuvant for improving the effect of these drugs in treating diabetic kidney disease.

Accordingly, in a second aspect, the present invention relates to a pharmaceutical composition for enhancing the efficacy of an antihypertensive agent and/or an antidiabetic agent in preventing, alleviating, or treating diabetic kidney disease, the composition comprising a zinc salt and cyclo-hispro or a pharmaceutically acceptable salt thereof as active ingredients.

According to one embodiment of the present invention, there is provided a pharmaceutical composition for enhancing the efficacy of an ARB and/or an SGLT2 inhibitor in the treatment of diabetic kidney disease, the composition comprising, as active ingredients, a zinc salt; and cyclo-hispro or a pharmaceutically acceptable salt thereof.

The description of the constitution and effects of the zinc salt and cyclo-His-Pro, which are included as active ingredients in the pharmaceutical composition for enhancing the efficacy of an ARB and/or an SGLT2 inhibitor in the treatment of diabetic kidney disease according to the present invention, is the same as described above, and thus is omitted.

In addition, the types of antihypertensive and antidiabetic agents (specifically, ARB and SGLT2) that can exhibit a synergistic effect when combined with the zinc salt and cyclo-hispro are the same as described above, and thus, the description thereof is omitted.

In relation to the second aspect, the present invention provides a method for improving the effect of preventing, alleviating, or treating diabetic kidney disease, the method comprising administering to a subject in need thereof an effective amount of a composition comprising a zinc salt and cyclo-hispro or a pharmaceutically acceptable salt thereof. Here, the subject is the same as described in the first aspect, and thus the description thereof is omitted. Alternatively, the subject may be a patient who exhibits, or is expected to exhibit, side effects due to overdose or long-term administration of an antihypertensive and/or antidiabetic agent. For example, the subject may be a patient with diabetic kidney disease who receives an antihypertensive and/or antidiabetic agent, preferably an ARB and/or an SGLT2 inhibitor.

In connection with the second aspect, the present invention further provides a use of a composition comprising a zinc salt; and cyclo-hispro or a pharmaceutically acceptable salt thereof, for use in enhancing the effect of preventing, alleviating, or treating diabetic kidney disease.

In connection with the second aspect, the present invention also provides a use of a composition comprising a zinc salt; and cyclo-hispro or a pharmaceutically acceptable salt thereof, for the manufacture of a medicament for enhancing the effect of preventing, alleviating, or treating diabetic kidney disease.

In the present invention, the composition comprising the zinc salt and cyclo-hispro or a pharmaceutically acceptable salt thereof may be administered in combination with an antihypertensive and/or antidiabetic agent for the purpose of enhancing the efficacy of the antihypertensive and/or antidiabetic agents in preventing, alleviating, or treating diabetic kidney disease. Here, the description of the antihypertensive agent and the antidiabetic agent are the same as described in the first aspect, and thus is omitted.

In a third aspect, the present invention relates to a health functional food composition for preventing or alleviating diabetic kidney disease, the composition comprising, as active ingredients, a zinc salt; and cyclo-hispro or a food-acceptable salt thereof.

In connection with the third aspect, the present invention also provides a use of a composition comprising a zinc salt; and cyclo-hispro or a food-acceptable salt thereof, for the manufacture of a health functional food for enhancing the effect of preventing, alleviating, or treating diabetic kidney disease.

In the present invention, the composition comprising the zinc salt and cyclo-hispro or a food-acceptable salt thereof may be co-administered to a diabetic kidney disease patient receiving an antihypertensive and/or antidiabetic agent for the purpose of enhancing the efficacy of the antihypertensive and/or antidiabetic agents in preventing, alleviating, or treating diabetic kidney disease. Here, the description of the antihypertensive agent and the antidiabetic agent are the same as described in the first aspect, and thus is omitted.

According to one embodiment of the present invention, the health functional food composition may be for enhancing the effect of alleviating diabetic kidney disease in a diabetic kidney disease patient receiving an antihypertensive and/or antidiabetic agent, for example, an ARB and/or an SGLT2 inhibitor.

In the health functional food composition of the present invention, the description of the constitution and effects of the zinc salt and cyclo-His-Pro which are included as active ingredients is the same as described above, and thus is omitted.

In the present invention, the term "food-acceptable salt" includes a salt derived from a food-acceptable organic acid, inorganic acid or base. The types of the food-acceptable salts are the same as the types of "pharmaceutically acceptable salts" described in the first aspect above, and thus the description thereof is omitted.

In the present invention, the term "health functional food" includes both the meanings of "functional food" and "health food."

In the present invention, the term "functional food" is the same term as food for special health use (FoSHU), and refers to a food with high medicinal and medical effects processed so that the bioregulatory function is efficiently exhibited in addition to nutritional supply.

In the present invention, the term "health food" refers to a food that has a more active health maintenance or promotion effect compared to general food, and health supplement food refers to a food for the purpose of health supplementation. The terms "functional food," "health food," and "health supplement food" may be used interchangeably. The food may be manufactured in various forms such as tablets, capsules, powders, granules, liquids, and pills to obtain useful effects in preventing or alleviating diabetic kidney disease.

As a specific example of such functional foods, the zinc salt and cyclo-hispro or a food-acceptable salt thereof according to the present invention can may be used to manufacture processed foods that are modified to preserve the characteristics of agricultural products, livestock products or marine products while improving their storage properties.

The health functional food composition of the present invention may also be manufactured in the form of a nutritional supplement, food additive, feed, etc., and is intended for consumption by humans or animals including livestock.

The above type of food composition may be manufactured in various forms according to conventional methods known in the art. General foods, including but not limited to, beverages (including alcoholic beverages), fruits and their processed foods (e.g., canned fruits, bottled fruits, jams, marmalades, etc.), fish, meats and their processed foods (e.g., ham, sausages, corned beef, etc.), breads and noodles (e.g., udon, buckwheat noodles, ramen, spaghetti, macaroni, etc.), fruit juices, various drinks, cookies, taffy, dairy products (e.g., butter, cheese, etc.), edible vegetable oils, margarine, vegetable proteins, retort foods, frozen foods, and various seasonings (e.g., soybean paste, soy sauce, sauces, etc.), may be produced by adding the zinc salt and cyclo-hispro or a food-acceptable salt thereof according to the present invention thereto.

In addition, nutritional supplements may be manufacture by adding the zinc salt and cyclo-hispro or a food-acceptable salt thereof of the present invention to capsules, tablets, pills, etc., but are not limited thereto.

In addition, health functional foods may be consumed, for example, by liquefying, granulating, encapsulating, or powdering the zinc salt and cyclo-hispro or a food-acceptable salt thereof of the present invention so that they can be manufactured in the form of tea, juice and drinks for drinking (health drinks), but are not limited thereto. In addition, in order to use the zinc salt and cyclo-hispro or a food-acceptable salt thereof of the present invention in the form of a food additive, they can be manufactured and used in the form of a powder or concentrate. In addition, the zinc salt and cyclo-hispro or a food-grade acceptable salt thereof of the present invention may be prepared in the form of a composition by mixing them with a known active ingredient known to be effective in preventing or alleviating diabetic kidney disease.

When the food composition of the present invention is used as a health beverage composition, the health beverage composition may contain various flavoring agents or natural carbohydrates as additional ingredients, as in conventional beverages. The natural carbohydrates mentioned above may be monosaccharides such as glucose and fructose; disaccharides such as maltose and sucrose; polysaccharides such as dextrin and cyclodextrin; and sugar alcohols such as xylitol, sorbitol and erythritol. Sweeteners may be natural sweeteners such as thaumatin and stevia extract; and synthetic sweeteners such as saccharin and aspartame. The proportion of the above natural carbohydrate is generally about 0.01 to 0.04 g, preferably about 0.02 to 0.03 g, per 100 mL of the composition of the present invention.

The zinc salt and cyclo-hispro or a food-acceptable salt thereof of the present invention may be contained as an effective ingredient of a health functional food composition for preventing or alleviating diabetic kidney disease, and the amount thereof is an amount effective to obtain the above-mentioned preventive or alleviating effect, and is preferably, for example, 0.01 to 100 wt% based on the total weight of the entire composition, but is not particularly limited thereto. The health functional food composition of the present invention may be prepared in the form of a composition by mixing the zinc salt and cyclo-hispro or a food-grade acceptable salt thereof with a known active ingredient known to be effective in preventing or alleviating diabetic kidney disease.

In addition to the above, the health functional food of the present invention may contain various nutrients, vitamins, electrolytes, flavoring agents, coloring agents, pectic acid, salts of pectic acid, alginic acid, salts of alginic acid, organic acids, protective colloid thickeners, pH adjusters, stabilizers, preservatives, glycerin, alcohol, or carbonating agents. In addition, the health food of the present invention may contain fruit pulp for the production of natural fruit juice, fruit juice beverage, or vegetable beverage. These ingredients may be used independently or in combination. The proportion of these additives is not particularly important, but is typically selected to be in the range of 0.01 to 0.1 parts by weight per 100 parts by weight of the composition of the present invention.

### [MODES FOR CARRYING OUT THE INVENTION]

Hereinafter, the present invention will be described in more detail by way of examples. These examples are only for illustrating the present invention, and it will be obvious to those skilled in the art that the scope of the present invention is not interpreted as limited by these examples.

### [Example 1]

### Confirmation of the Effects of CycloZ, ARB/ACE Inhibitor and/or SGLT2 Inhibitor Administration on Reducing Albuminuria and Albuminuria/Creatinine Ratio in a Diabetic Animal Model.

### 1-1. Experimental Animals and Administered Drugs

Five-week-old KKay mice were purchased from Clea, Japan, and as a normal control group, five-week-old C57BL6/J mice were purchased from Daehan Bio Link Co., Ltd. Breeding was performed under constant conditions (temperature: 22±2 °C, relative humidity: 55±10%, circadian cycle: 12 hours), and Purina mouse diet was used as feed and distilled water was used as drinking water. Mice were used in the experiment after a one-week adaptation period following purchase. Lorsartan used in the examples below was purchased from Angene, and dapagliflozin was purchased from Betapahrma. Metabolic cages were prepared using products from Jeongdo B&P Co., Ltd. Isoflurane, which is necessary for anesthesia and dissection of mice, was purchased from Hana Pharm Co., Ltd., and the RC2 Rodent Circuit Controller Anesthesia System from Vetequip was prepared. Phosphate buffered saline (PBS) was purchased from Hyclone.

The KKAy animal model is known to have 10-50 times higher proteinuria (albuminuria) than the db/db animal model, and is reported to show the most severe mesangial matrix expansion among all models. Five-week-old KKAy mice purchased from Clea Japan were pre-bred for one week and then divided into groups as shown in Table 1. Losartan was dissolved in drinking water to allow a daily intake of 20 mg/kg per subject, while dapagliflozin (2 mg/kg), CycloZ (15 mg/kg), or combinations thereof were orally administered daily. The normal control group and the negative control group were orally administered the same amount of distilled water daily.

**[Table 1]**

| **Group** | **Administered drug** |
|---|---|
| Normal control group | Distilled water |
| Negative control group | Distilled water |
| Group 1 | Losartan 20 mg/kg |
| Group 2 | Dapagliflozin 2 mg/kg |
| Group 3 | CycloZ 15 mg/kg(CHP 5 mg/kg + Zn 10 mg/kg) |
| Group 4 | Losartan 20 mg/kg + CycloZ 15 mg/kg (CHP 5 mg/kg + Zn 10 mg/kg) |
| Group 5 | Dapagliflozin 2 mg/kg + CycloZ 15 mg/kg (CHP 5 mg/kg + Zn 10 mg/kg) |
| Group 6 | Losartan 20 mg/kg + dapagliflozin 2 mg/kg |
| Group 7 | Losartan 20 mg/kg + dapagliflozin 2 mg/kg + CycloZ 15 mg/kg (CHP 5 mg/kg + Zn 10 mg/kg) |

### 1-2. Measurement of Urine Albumin

At week 16 of administration, 24-hour urine was collected using a metabolic cage and centrifuged at 2,000 rpm for 10 minutes to remove suspended solids. Albumin was measured with a DCA vantage analyzer (Siemens) and DCA^{®} Microalbumin/Creatinine Urine Test cartridge (Siemens) using urine diluted 1/10 with distilled water.

As a result, as shown in FIG. 1A, albuminuria (mg/dL) was detected as 51.4 in the negative control group, 39.3 in the Losartan-alone administration group (Group 1), 45.4 in the CycloZ-alone administration group (Group 3), and 11.1 in the Losartan + CycloZ combination administration group (Group 4), and each drug administration group showed a 23.5%, 11.7%, and 78.4% decrease in albuminuria compared to the negative control group. From the above results, it was confirmed that the combined administration of Losartan + CycloZ showed a synergistic effect in reducing albuminuria.

In addition, as shown in FIG. 1B, albuminuria (mg/dL) was detected as 51.4 in the negative control group, 31.1 in the Dapagliflozin-alone administration group (group 2), 45.4 in the CycloZ-alone administration group (group 3), and 22.4 in the Dapagliflozin + CycloZ combination administration group (group 5), and each drug administration group showed a decrease of 39.5%, 11.7%, and 56.4% compared to the negative control group. From the above results, it was confirmed that the combined administration of Dapagliflozin + CycloZ showed a synergistic effect in reducing albuminuria.

In addition, as shown in FIG. 1C, albuminuria (mg/dL) was detected as 51.4 in the negative control group, 45.4 in the CycloZ-alone administration group (group 3), 6.7 in the Losartan + Dapagliflozin combination administration group (group 6), and 2.2 in the Losartan + Dapagliflozin + CycloZ combination administration group (group 7), and each drug administration group showed a decrease of 11.7%, 87%, and 95.7% compared to the negative control group. From the above results, it was confirmed that the combined administration of Losartan + Dapagliflozin + CycloZ showed a synergistic effect in reducing albuminuria.

Lastly, FIG. 1D shows the results of comparing all albuminuria measurements of groups 1 to 7, wherein albuminuria (mg/dL) was detected as 51.4 in the negative control group, 39.3 in the Losartan-alone administration group (group 1), 31.1 in the Dapagliflozin-alone administration group (group 2), 45.4 in the CycloZ-alone administration group (group 3), 11.1 in the Losartan + CycloZ combination administration group (group 4), 22.4 in the Dapagliflozin + CycloZ combination administration group (group 5), 6.7 in the Losartan + Dapagliflozin combination administration group (group 6), and 2.2 in the Losartan + Dapagliflozin + CycloZ combination administration group (group 7), and each drug administration group showed a decrease of 23.5%, 39.5%, 11.7%, 56.4% 78.4%, 87%, and 95.7% compared to the negative control group. In particular, it was confirmed that the combined administration of Losartan + Dapagliflozin + CycloZ showed a significant albuminuria reduction effect compared to the groups administered alone or in combination of the two drugs, and thus, the synergistic effect of the three combined administrations was the most excellent.

### 1-3. Confirmation of Albuminuria/Creatinine Ratio

Creatinine was measured using undiluted urine and a mouse creatinine assay kit (Crystalchem). The measured albumin (mg/dL) value was divided by the creatinine (g/dL) value to derive the ACR (mg/g) value.

As a result, as shown in FIG. 2A, it was confirmed that the ACR value of the Losartan + CycloZ combination administration group (Group 4) decreased more than that of the groups administered Losartan or CycloZ alone (Group 1 or Group 3), demonstrating a synergistic effect due to the combination administration.

In addition, as shown in FIG. 2B, it was confirmed that the ACR value of the Dapagliflozin + CycloZ combination administration group (Group 5) decreased more than that of the groups administered Dapagliflozin or CycloZ alone (Group 2 or Group 3), demonstrating a synergistic effect due to the combination administration.

Finally, as shown in FIG. 2C, it was confirmed that the ACR value of the Losartan + Dapagliflozin + CycloZ combination administration group (Group 7) was significantly reduced compared to the groups administered Losartan, Dapagliflozin, or CycloZ alone (Groups 1 to 3), and was further reduced compared to the Losartan + Dapagliflozin combination administration group.

### [Example 2]

### Confirmation of the Effect of Increasing Glomerular Filtration Rate in a Diabetic Animal Model

At week 17 of administration, the hair on the back of the mouse was shaved with a hair caliper, Nair hair removal cream was applied, and after 5 minutes, the hair was removed by wiping it off with a gauze soaked in warm water. The next day, a GFR monitoring device (Medibeacon) was attached to the back of the mouse, and FITC-sinistrin (Medibeacon) dissolved in physiological saline was injected into the tail vein at a concentration of 150 mg/kg. After real-time measurement for 90 minutes, the device was removed and the GFR values were analyzed using Medibeacon Studio software.

As a result, as shown in Table 2, the GFR value of the Losartan + Dapagliflozin + CycloZ combination administration group (Group 7) was significantly increased compared to the negative control group, and was further increased compared to the Losartan + CycloZ combination administration group (Group 4) and the Dapagliflozin + CycloZ combination administration group (Group 5).

**[Table 2]**

| **Group** | **GFR** |
|---|---|
| Negative control group | 0.580 |
| Losartan + CycloZ | 0.839 |
| Dapagliflozin + CycloZ | 0.826 |
| Losartan + Dapagliflozin + CycloZ | 0.932 |

### [Example 3]

### Confirmation of the Effects of CycloZ and/or DPP-4 Inhibitor Administration on Reducing Albuminuria/Creatinine Ratio in a Diabetic Animal Model.

### 3-1. Administration of Drugs for Mild Condition

Five-week-old KKAy mice of Example 1-1 were pre-bred for one week and then divided into groups as shown in Table 1 so that the average body weights were the same. The control group was orally administered distilled water for 11 weeks, while Group 1 was orally administered 15 mg/kg of CycloZ, Group 2 was orally administered 10 mg/kg of sitagliptin, and Group 3 was orally administered 10 mg/kg of sitagliptin + 15 mg/kg of CycloZ daily for the same period.

**[Table 3]**

| **Group** | **Administered drug** |
|---|---|
| Negative control group (CTRL) | Distilled water |
| Group 1 | CycloZ 15 mg/kg |
| Group 2 | Sitagliptin 10 mg/kg |
| Group 3 | Sitagliptin 10 mg/kg + CycloZ 15 mg/kg |

### 3-2. Confirmation of Albuminuria/Creatinine Ratio in Mild Condition

At week 11 of administration, 24-hour urine was collected using a metabolic cage and centrifuged at 1,000 rpm for 5 minutes to remove suspended solids. Albumin was measured with a DCA vantage analyzer (Siemens) and DCA^{®} Microalbumin/Creatinine Urine Test cartridge (Siemens) using urine diluted 1/2 with distilled water. Creatinine was measured using undiluted urine and a mouse creatinine assay kit (Crystalchem). The measured albumin (mg/dL) value was divided by the creatinine (g/dL) value to derive the ACR (mg/g) value.

As a result, as confirmed in FIG. 3A, the ACR (mg/g) value was 6,379 mg/g in the negative control group, 4,042 mg/g in the CycloZ-alone administration group, 4,852 mg/g in the Sitagliptin-alone administration group, and 2,378 mg/g in the Sitagliptin + CycloZ combination administration group. These values were reduced by approximately 37%, 24%, and 63%, respectively, compared to the control group, and it was confirmed that the ACR values of the Sitagliptin + CycloZ combination administration group showed a significantly improved ACR reduction effect compared to other single administration groups, showing a synergistic effect by CycloZ.

### 3-3. Administration of Drugs for Severe Condition

After breeding five-week-old KKAy mice of Example 1-1 for 6 weeks, urine was collected for 24 hours and ACR was measured using the same method as in Example 3-2. The groups were randomly divided as shown in Table 4 so that the average ACR values were the same, and then the drug was orally administered daily for 11 weeks from the age of 12 weeks as shown in Table 4.

**[Table 4]**

| **Group** | **Administered drug** |
|---|---|
| Negative control group (CTRL) | Distilled water |
| Group 1 | CycloZ 15 mg/kg |
| Group 2 | Sitagliptin 10 mg/kg |
| Group 3 | Sitagliptin 10 mg/kg + CycloZ 15 mg/kg |

### 3-4. Confirmation of Albuminuria/Creatinine Ratio in Severe Condition

At week 11 of administration, 24-hour urine was collected using a metabolic cage, and ACR was measured using the same method as in Example 3-2.

As a result, as confirmed in FIG. 3B, the ACR (mg/g) value was 4,450 mg/g in the negative control group, 4,701 mg/g in the CycloZ-alone administration group, 2,301 mg/g in the Sitagliptin-alone administration group, and 1,591 mg/g in the Sitagliptin + CycloZ combination administration group. In administration from the severe stage, CycloZ-alone administration did not show a significant effect on improving ACR, but the Sitagliptin-alone administration group showed a 48% decrease compared to the negative control group, and the Sitagliptin + CycloZ combination administration group showed a 31% decrease compared to the Sitagliptin-alone administration group and a 64% decrease compared to the negative control group. This could confirm the synergistic effect of the combined administration of Sitagliptin and CycloZ on alleviating severe diabetic kidney disease.

### [Example 4]

### Confirmation of the Effects of CycloZ, ARB/ACE inhibitor, and/or DPP-4 inhibitor administration on Reducing Albuminuria/Creatinine Ratio in a Diabetic Animal Model.

### 4-1. Administration of Drugs for Severe Condition

After breeding five-week-old KKAy mice of Example 1-1 for 6 weeks, urine was collected for 24 hours and ACR was measured using the same method as in Example 3-2. The groups were randomly divided as shown in Table 5 so that the average ACR values were the same, and then the drug was orally administered daily for 9 weeks from the age of 12 weeks as shown in Table 5. At this time, losartan was dissolved in drinking water to allow a daily intake of 20 mg/kg per subject, and the remaining drugs were orally administered daily.

**[Table 5]**

| **Group** | **Administered drug** |
|---|---|
| Negative control group (CTRL) | Distilled water |
| Group 1 | CycloZ 15 mg/kg |
| Group 2 | Losartan 20 mg/kg |
| Group 3 | Losartan 20 mg/kg + CycloZ 15 mg/kg |
| Group 4 | Losartan 20 mg/kg + Sitagliptin 10 mg/kg |
| Group 5 | Losartan 20 mg/kg + Sitagliptin 10 mg/kg + CycloZ 15 mg/kg |

### 4-2. Confirmation of Albuminuria/Creatinine Ratio in Severe Condition

At week 9 of administration, 24-hour urine was collected using a metabolic cage, and ACR was measured using the same method as in Example 3-2.

As a result, as confirmed in FIG. 4, the ACR (mg/g) value was 7,118 mg/g in the negative control group, 6,639 mg/g in the CycloZ-alone administration group, 7,158 mg/g in the Losartan-alone administration group, and 4,125 mg/g in the Losartan + CycloZ combination administration group. In administration from the severe stage, CycloZ-alone and Losartan-alone administration did not show a significant effect on alleviating ACR, but the Losartan + CycloZ combination administration group showed a 42% decrease compared to the negative control group. This could confirm the synergistic effect of the combined administration of Losartan + CycloZ on alleviating severe diabetic kidney disease.

In addition, as confirmed in FIG. 4, the ACR value of the Losartan + Sitagliptin combination administration group was 2,768 mg/g, and the Losartan + Sitagliptin + CycloZ combination administration group was 2,025 mg/g, wherein these values were reduced by 61.1% and 71.6%, respectively, compared to the negative control group. The ACR value of the Losartan + Sitagliptin + CycloZ combination administration group was reduced more than that of the Losartan + Sitagliptin combination administration group, which confirmed that there was a synergistic effect due to CycloZ.

### [Example 5]

### Confirmation of the Effects of CycloZ and/or Biguanide Drug Administration on Reducing Albuminuria/Creatinine Ratio in a Diabetic Animal Model.

### 5-1. Administration of Drugs for Severe Condition

After breeding five-week-old KKAy mice of Example 1-1 for 6 weeks, urine was collected for 24 hours and ACR was measured using the same method as in Example 3-2. The groups were randomly divided as shown in Table 6 so that the average ACR values were the same, and then the drug was orally administered daily for 12 weeks from the age of 12 weeks as shown in Table 6.

**[Table 6]**

| **Group** | **Administered drug** |
|---|---|
| Negative control group (CTRL) | Distilled water |
| Group 1 | CycloZ 15 mg/kg |
| Group 2 | Metformin 200 mg/kg |
| Group 3 | Metformin 200 mg/kg + CycloZ 15 mg/kg |

### 5-2. Confirmation of Albuminuria/Creatinine Ratio in Severe Condition

At week 12 of administration, 24-hour urine was collected using a metabolic cage, and ACR was measured using the same method as in Example 3-2.

As a result, as confirmed in FIG. 5, the ACR (mg/g) value was 2,369 mg/g in the negative control group, 2,792 mg/g in the CycloZ-alone administration group, 2,952 mg/g in the Metformin-alone administration group, and 1,117 mg/g in the Metformin + CycloZ combination administration group. In administration from the severe stage, CycloZ-alone and Metformin-alone administration did not show a significant effect on alleviating ACR, but the Metformin + CycloZ combination administration group showed a 53% decrease compared to the negative control group. This could confirm the synergistic effect of the combined administration of Metformin + CycloZ on alleviating severe diabetic kidney disease.

### [Example 6]

### Confirmation of the Effects of Reducing Albuminuria/Creatinine Ratio by a Combination of Zinc salt and CHP in a Diabetic Animal Model.

The same animal model as in Example 1 was pre-bred for one week and then divided into groups as shown in Table 7. CHP (5 mg/kg or 35 mg/kg), CycloZ (15 mg/kg), and Bardoxolone methyl (mg/kg) were orally administered daily, respectively. The normal control group and the negative control group were orally administered the same amount of distilled water daily.

**[Table 7]**

| **Group** | **Administered drug** |
|---|---|
| Normal control group | Distilled water |
| Negative control group | Distilled water |
| Group 1 | CHP 5 mg/kg |
| Group 2 | CHP 35 mg/kg |
| Group 3 | CycloZ 15 mg/kg(CHP 5 mg/kg + Zn 10 mg/kg) |
| Positive control group | Bardoxolone methyl (10 mg/kg) |

Creatinine was measured using undiluted urine and a mouse creatinine assay kit (Crystalchem). The measured albumin (mg/dL) value was divided by the creatinine (g/dL) value to derive the ACR (mg/g) value. As a result, as shown in FIG. 6, the ACR value of the CycloZ administration group (group 3) was significantly reduced compared to the groups administered CHP alone (group 1 or group 2), demonstrating a synergistic effect due to the combined administration of CHP and Zn compared to the administration of CHP alone.

Although the specific contents of the present invention have been described in detail above, it will be clear to those skilled in the art that these specific descriptions are merely preferred embodiments, and do not limit the scope of the present invention. Accordingly, it can be said that the substantial scope of the present invention is defined by the appended claims and their equivalents.

## Claims

1. A pharmaceutical composition for preventing or treating diabetic kidney disease, the composition comprising, as active ingredients:
a zinc salt; and
cyclo-hispro or a pharmaceutically acceptable salt thereof.

2. The pharmaceutical composition of claim 1, further comprising, as an additional active ingredient, an antihypertensive agent selected from the group consisting of angiotensin II receptor blockers and angiotensin-converting-enzyme inhibitors.

3. The pharmaceutical composition of claim 1, further comprising, as an additional active ingredient, an antidiabetic agent selected from the group consisting of sodium glucose cotransporter 2 inhibitors, dipeptidyl peptidase-4 inhibitors, and biguanide drugs.

4. The pharmaceutical composition of claim 1, further comprising, as additional active ingredients, an antihypertensive agent selected from the group consisting of angiotensin II receptor blockers and angiotensin-converting-enzyme inhibitors; and an antidiabetic agent selected from the group consisting of sodium glucose cotransporter 2 inhibitors, dipeptidyl peptidase-4 inhibitors, and biguanide drugs.

5. The pharmaceutical composition of claim 2 or claim 4, wherein the angiotensin II receptor blocker is any one selected from the group consisting of losartan, valsartan, candesartan, olmesartan, telmisartan, fimasartan, irbesartan, eprosartan, and azilsartan, and
the angiotensin-converting-enzyme inhibitor is any one selected from the group consisting of captopril, zofenopril, enalapril, ramipril, quinapril, perindopril, lisinopril, benazepril, imidapril, trandolapril, cilazapril, and fosinopril.

6. The pharmaceutical composition of claim 3 or claim 4, wherein the sodium glucose cotransporter 2 inhibitor is any one selected from the group consisting of dapagliflozin, canagliflozin, empagliflozin, ipragliflozin, tofogliflozin, luseogliflozin, remogliflozin, remogliflozin etabonate, and ertugliflozin,
the dipeptidyl peptidase-4 inhibitor is any one selected from the group consisting of sitagliptin, linagliptin, vildagliptin, gemigliptin, saxagliptin, alogliptin, teneligliptin, anagliptin, and evogliptin, and
the biguanide drug is any one selected from the group consisting of metformin, buformin, and phenformin.

7. The pharmaceutical composition of claim 1, wherein the pharmaceutical composition exhibits at least one effect selected from the group consisting of the following i) to iii):
i) reducing albuminuria;
ii) reducing albuminuria/creatinine ratio; and
iii) increasing glomerular filtration rate.

8. The pharmaceutical composition of claim 1, wherein the pharmaceutical composition is administered simultaneously, separately, or sequentially with an antihypertensive agent and/or an antidiabetic agent,
wherein the antihypertensive agent is any one selected from the group consisting of angiotensin II receptor blockers and angiotensin-converting-enzyme inhibitors; and
the antidiabetic agent is any one selected from the group consisting of sodium glucose cotransporter 2 inhibitors, dipeptidyl peptidase-4 inhibitors, and biguanide drugs.

9. A pharmaceutical composition for enhancing the efficacy of an antihypertensive agent and/or an antidiabetic agent in the treatment of diabetic kidney disease, the composition comprising, as active ingredients:
a zinc salt; and
cyclo-hispro or a pharmaceutically acceptable salt thereof.

10. The pharmaceutical composition of claim 9, wherein the antihypertensive agent is an angiotensin II receptor blocker selected from the group consisting of losartan, valsartan, candesartan, olmesartan, telmisartan, fimasartan, irbesartan, eprosartan, and azilsartan; or
an angiotensin-converting-enzyme inhibitor selected from the group consisting of captopril, zofenopril, enalapril, ramipril, quinapril, perindopril, lisinopril, benazepril, imidapril, trandolapril, cilazapril, and fosinopril.

11. The pharmaceutical composition of claim 9, wherein the antidiabetic agent is a sodium glucose cotransporter 2 inhibitor selected from the group consisting of dapagliflozin, canagliflozin, empagliflozin, ipragliflozin, tofogliflozin, luseogliflozin, remogliflozin, remogliflozin etabonate, and ertugliflozin;
a dipeptidyl peptidase-4 inhibitor selected from the group consisting of sitagliptin, linagliptin, vildagliptin, gemigliptin, saxagliptin, alogliptin, teneligliptin, anagliptin, and evogliptin; or
a biguanide drug selected from the group consisting of metformin, buformin, and phenformin.

12. The pharmaceutical composition of claim 9, wherein the pharmaceutical composition exhibits at least one effect selected from the group consisting of the following i) to iii):
i) reducing albuminuria;
ii) reducing albuminuria/creatinine ratio; and
iii) increasing glomerular filtration rate.

13. The pharmaceutical composition of claim 9, wherein the pharmaceutical composition is administered simultaneously, separately, or sequentially with an antihypertensive agent and/or an antidiabetic agent,
wherein the antihypertensive agent is an angiotensin II receptor blocker selected from the group consisting of losartan, valsartan, candesartan, olmesartan, telmisartan, fimasartan, irbesartan, eprosartan, and azilsartan; or an angiotensin-converting-enzyme inhibitor selected from the group consisting of captopril, zofenopril, enalapril, ramipril, quinapril, perindopril, lisinopril, benazepril, imidapril, trandolapril, cilazapril, and fosinopril, and
the antidiabetic agent is a sodium glucose cotransporter-2 inhibitor selected from the group consisting of dapagliflozin, canagliflozin, empagliflozin, ipragliflozin, tofogliflozin, luseogliflozin, remogliflozin, remogliflozin etabonate, and ertugliflozin; a dipeptidyl peptidase-4 inhibitor selected from the group consisting of sitagliptin, linagliptin, vildagliptin, gemigliptin, saxagliptin, alogliptin, teneligliptin, anagliptin, and evogliptin; or a biguanide drug selected from the group consisting of metformin, buformin, and phenformin.

14. A health functional food composition for preventing or alleviating diabetic kidney disease, the composition comprising, as active ingredients:
a zinc salt; and
cyclo-hispro or a food-acceptable salt thereof.

15. The health functional food composition of claim 14, wherein the health functional food composition is for enhancing the effect of alleviating diabetic kidney disease in a DKD patient receiving an antihypertensive agent and/or an antidiabetic agent,
wherein the antihypertensive agent is an angiotensin II receptor blocker selected from the group consisting of losartan, valsartan, candesartan, olmesartan, telmisartan, fimasartan, irbesartan, eprosartan, and azilsartan; or an angiotensin-converting-enzyme inhibitor selected from the group consisting of captopril, zofenopril, enalapril, ramipril, quinapril, perindopril, lisinopril, benazepril, imidapril, trandolapril, cilazapril, and fosinopril, and
the antidiabetic agent is a sodium glucose cotransporter-2 inhibitor selected from the group consisting of dapagliflozin, canagliflozin, empagliflozin, ipragliflozin, tofogliflozin, luseogliflozin, remogliflozin, remogliflozin etabonate, and ertugliflozin; a dipeptidyl peptidase-4 inhibitor selected from the group consisting of sitagliptin, linagliptin, vildagliptin, gemigliptin, saxagliptin, alogliptin, teneligliptin, anagliptin, and evogliptin; or a biguanide drug selected from the group consisting of metformin, buformin, and phenformin.

16. The health functional food composition of claim 14, wherein the health functional food composition exhibits at least one effect selected from the group consisting of the following i) to iii):
i) reducing albuminuria;
ii) reducing albuminuria/creatinine ratio; and
iii) increasing glomerular filtration rate.

17. The health functional food composition of claim 14, wherein the health functional food composition is administered simultaneously, separately, or sequentially with an antihypertensive agent and/or an antidiabetic agent,
wherein the antihypertensive agent is an angiotensin II receptor blocker selected from the group consisting of losartan, valsartan, candesartan, olmesartan, telmisartan, fimasartan, irbesartan, eprosartan, and azilsartan; or an angiotensin-converting-enzyme inhibitor selected from the group consisting of captopril, zofenopril, enalapril, ramipril, quinapril, perindopril, lisinopril, benazepril, imidapril, trandolapril, cilazapril, and fosinopril, and
the antidiabetic agent is a sodium glucose cotransporter-2 inhibitor selected from the group consisting of dapagliflozin, canagliflozin, empagliflozin, ipragliflozin, tofogliflozin, luseogliflozin, remogliflozin, remogliflozin etabonate, and ertugliflozin; a dipeptidyl peptidase-4 inhibitor selected from the group consisting of sitagliptin, linagliptin, vildagliptin, gemigliptin, saxagliptin, alogliptin, teneligliptin, anagliptin, and evogliptin; or a biguanide drug selected from the group consisting of metformin, buformin, and phenformin.

18. A method for preventing, alleviating, or treating diabetic kidney disease, the method comprising administering to a subject in need thereof an effective amount of a composition comprising a zinc salt and cyclo-hispro or a pharmaceutically acceptable salt thereof.

19. A method for enhancing the efficacy of an antihypertensive and/or an antidiabetic agent in the treatment of diabetic kidney disease, the method comprising administering to a subject in need thereof an effective amount of a composition comprising a zinc salt and cyclo-hispro or a pharmaceutically acceptable salt thereof.

20. A use of a composition comprising a zinc salt and cyclo-hispro or a pharmaceutically acceptable salt thereof for the manufacture of a medicament for preventing, alleviating, or treating diabetic kidney disease.

21. A use of a composition comprising a zinc salt and cyclo-hispro or a pharmaceutically acceptable salt thereof for the manufacture of a medicament for enhancing the efficacy of an antihypertensive agent and/or an antidiabetic agent in preventing, alleviating, or treating diabetic kidney disease.
